Europäisches Patentamt

European Patent Office

Office européen des brevets

(11) Numéro de publication: **0 421 878 A1**

# (12) DEMANDE DE BREVET EUROPEEN

(21) Numéro de dépôt: 90402749.7

(22) Date de dépôt: **04.10.90**

(51) Int. Cl.5: **C07C 209/36**, C07C 211/52

(30) Priorité: 04.10.89 FR 8912958

(43) Date de publication de la demande:
**10.04.91 Bulletin 91/15**

(84) Etats contractants désignés:
**AT BE CH DE DK ES FR GB GR IT LI LU NL SE**

(71) Demandeur: **RHONE-POULENC CHIMIE**
**25, quai Paul Doumer**
**F-92408 Courbevoie Cédex(FR)**

(72) Inventeur: **Grosselin, Jean-Michel**
**12 Allée des Erables**
**F-69340 Francheville(FR)**
Inventeur: **Bailliard, Rose-Marie**
**5, rue Molière**
**F-69006 Lyon(FR)**
Inventeur: **Cordier, Georges**
**50, Chemin des Hermières**
**F-69340 Francheville(FR)**

(74) Mandataire: **Ricalens, François et al**
**RHONE-POULENC CHIMIE Service Brevets**
**Chimie 25, quai Paul Doumer**
**F-92408 Courbevoie(FR)**

(54) **Procédé d'hydrogénation de dérivés halogéno-nitroaromatiques en présence de catalyseurs à base de métaux nobles.**

(57) La présente invention concerne un procédé d'hydrogénation de composés nitrés et halogénés aromatiques. Elle concerne plus particulièrement la préparation d'amines halogénées.

La présente invention a su à partir du platine ou du palladium, le déposer sur un support à base d'alumine, ce qui rend ce catalyseur d'hydrogénation aisément décantable donc recyclable, et lui apporte toutes les qualités requises pour l'hydrogénation des dérivés halogéno nitroaromatiques.

EP 0 421 878 A1

# PROCEDE D'HYDROGENATION DE DERIVES HALOGENO NITROAROMATIQUES EN PRESENCE DE CATALYSEURS A BASE DE METAUX NOBLES

La présente invention concerne un procédé d'hydrogénation de composés nitrés et halogénés aromatiques. Elle concerne plus particulièrement la préparation d'amines halogénées.

Lors de la mise en oeuvre d'un procédé d'hydrogénation sur un dérivé aromatique nitré portant des atomes d'halogène liés au noyau aromatique, il se produit en même temps que la transformation du groupe nitro en groupe aminé un phénomène de déshalogénation avec perte des atomes d'halogène sous forme d'hydrohalogénures.

Ce phénomène est connu depuis très longtemps puisqu'il a été décrit en 1904 par P. Sabatier et A. Mailhe.

De nombreux brevets ont été déposés pour éviter cette réaction secondaire tout en permettant de conserver une activité correcte au catalyseur.

Ces brevets peuvent être divisés en deux groupes : ceux utilisant comme catalyseur d'hydrogénation le nickel de Raney et ceux utilisant le platine ou le palladium.

Tous ces brevets décrivent l'utilisation d'un catalyseur modifié.

Dans le premier groupe de brevets décrivant l'utilisation du nickel de Raney on peut citer les brevets US 3067253, GB 1191610, JP 73-49728, GB 1498722 et FR 2245615.

Le brevet US 3067253 décrit l'utilisation de nickel de Raney auquel est adjoint un hydroxyde de calcium ou de magnésium. Les températures réactionnelles sont néanmoins toujours basses (25 à 60° C) afin d'éviter la déshalogénation, ce qui ne permet pas de pouvoir utiliser ces procédés industriellement.

Le brevet GB 1191610 décrit l'utilisation du nickel de Raney associé à la présence d'un thiocyanate. Ce procédé ne permet qu'une hydrogénation lente (4 heures à 8 heures) et le catalyseur est altéré lors de l'hydrogénation, ce qui ne permet pas d'envisager un procédé en continu.

Le brevet JP 73-49728 décrit l'utilisation du nickel de Raney associé à la présence d'une alkylamine, d'une alkanolamine ou d'une base hétérocyclique. Dans ce brevet la température d'hydrogénation est limitée comme dans le brevet US précédemment mentionné à 60° C.

Cette température ne permet pas une exploitation industrielle satisfaisante car la productivité du procédé à ces températures est insuffisante. Il est même indiqué dans le brevet FR 2245615 que le procédé du brevet JP 73-49728 ne permet pas d'éviter la déshalogénation de façon satisfaisante puisque au moins 5 % de l'aniline obtenue est déshalogénée.

Le brevet GB 1498722 décrit l'utilisation de nickel de Raney avec un trialkylphosphite. La température de réaction est d'environ 100° C mais le taux de déshalogénation est très élevé puisqu'il varie entre 2 et 8 %. Ce procédé n'est donc pas envisageable industriellement.

Le dernier brevet décrivant l'utilisation du nickel de Raney est le brevet FR 2245615 qui l'associe avec un inhibiteur de deshalogénation choisi parmi le dicyandiamide, le cyanamide et le cyanamide calcique. La température de la réaction d'hydrogénation est comprise entre 50 et 130° C et les taux de déshalogénation sont toujours inférieurs à 0,15 %.

Dans le deuxième groupe de brevets décrivant l'utilisation de métaux de la mine du platine on peut citer les brevets FR 2330669 et FR 2127092.

Le brevet FR 2330669 décrit l'utilisation comme catalyseur d'hydrogénation de composés nitroaromatiques chlorés, du platine déposé sur charbon et inhibé par la présence d'un dérivé soufré choisi parmi les thioéthers et les disulfures. Le taux de déshalogénation est très faible (0,01 à 0,08 %), ce faible taux de déshalogénation provient en grande partie du platine qui même en l'absence de dérivé soufré, ne provoque pas de déshalogénation.

Le brevet FR 2127092 décrit la préparation d'un catalyseur au platine déposé sur charbon qui est sulfuré. La préparation de ce catalyseur consiste à procéder d'abord à une hydrogénation du catalyseur puis à sulfurer ensuite celui-ci par addition d'hydrogène sulfuré selon une quantité variant entre 0,45 et 0,55 mole d'hydrogène sulfuré par mole d'hydrogène absorbée.

D'une part la préparation du catalyseur est difficile, d'autre part l'utilisation d'un catalyseur déposé sur charbon ne permet pas une décantation facile de la masse catalytique et rend ainsi très difficile l'exploitation du procédé selon un mode continu. L'utilisation de platine, catalyseur très onéreux a fait reculer l'industrie quant à la mise en oeuvre industrielle d'un tel procédé.

La présente invention a su à partir du platine ou du palladium, le déposer sur un support à base d'alumine, ce qui rend ce catalyseur d'hydrogénation aisément décantable donc recyclable, et lui apporte toutes les qualités requises pour l'hydrogénation des dérivés halogéno nitroaromatiques.

Elle consiste à mettre en présence le dérivé halogéno nitroaromatique ou polyhalogéno nitroaromatique avec le catalyseur d'hydrogénation constitué de platine ou de palladium déposé sur un support à base d'alumine.

Les dérivés halogéno nitroaromatiques qui peuvent être hydrogénés selon le procédé de la présente invention répondent à la formule suivante :

$(Z)_q (Y)_p (X)_n - Ar - NO_2$

dans laquelle :

- Ar représente un radical mono, polycyclique ou hétérocyclique éventuellement substitué par un groupe alkyle contenant 1 à 4 atomes de carbone,
- X, Y, Z représentent chacun un halogène choisi parmi le fluor, le chlore et le brome,
- n, p, q représentent chacun un nombre entier supérieur ou égal à 0 et inférieur ou égal à 5, la somme n + p + q étant supérieure ou égale à 1.

On préfère utiliser un composé halogéno nitroaromatique monocyclique contenant un à trois atomes d'halogène choisi parmi le chlore et le fluor fixés sur le noyau et tout particulièrement l'invention s'applique aux dérivés suivants :

- les chloronitrobenzènes
- les fluoronitrobenzènes
- les dichloronitrobenzènes
- les monochloro monofluoro nitrobenzènes
- les trichloronitrobenzènes
- les chloronitrométhylbenzènes
- les fluoronitrométhylbenzènes

On effectue l'hydrogénation des dérivés halogéno-nitroaromatiques à l'aide du catalyseur selon l'invention dans les conditions usuelles d'hydrogénation. En raison du fort potentiel de non déshalogénation qu'apporte l'alumine, il est tout à fait possible de réaliser l'hydrogénation à une température comprise entre 60°C et 150°C. Elle est même réalisée préférentiellement entre 70°C et 110°C ce qui permet d'obtenir des productivités (quantité d'amine formée par heure et par volume de milieu réactionnel) comparatives avec les productivités des procédés visant la préparation d'amines non halogénées.

Un autre avantage du catalyseur selon l'invention que n'apportaient pas les catalyseurs déposés sur charbon est la facilité de sa mise en oeuvre par des procédés réalisés en continu. En effet la séparation des catalyseurs à base de métaux déposés sur alumine est nettement plus facile que celle des catalyseurs déposés sur charbon.

Un dernier avantage du catalyseur selon l'invention est la facilité de sa mise en oeuvre ; aucune préparation préalable du catalyseur n'est nécessaire comme dans le brevet FR 2127092 qui décrit une hydrogénation du catalyseur, suivie de sa sulfuration. Dans le présent procédé il est seulement nécessaire d'introduire tous les réactifs dans le réacteur d'hydrogénation : le métal déposé sur alumine, le dérivé halogéno-nitroaromatique, le solvant, puis de pressuriser le réacteur avec de l'hydrogène.

Le procédé de l'invention peut être mis en oeuvre en l'absence de solvant ou dans tout solvant inerte dans les conditions de la réaction tel que par exemple :

- l'eau
- les alcools tels que le méthanol, l'éthanol, l'isopropanol
- les solvants aromatiques tels que le toluène, le xylène.

On préfère utiliser le méthanol.

Pour une meilleure mise en oeuvre de l'invention, on préfère utiliser une quantité pondérale de catalyseur (platine ou palladium déposée sur alumine) par litre de milieu réactionnel comprise entre 1 g et 20 g. Dans le catalyseur, la quantité pondérale de métal calculée pour 100 grammes d'alumine est généralement comprise entre 0,1 g et 1 g.

Le procédé pouvant être mis en oeuvre en continu, la quantité de dérivé halogéno nitroaromatique ne peut être établie de manière statique mais sous forme de flux. Ainsi un débit d'environ 1 à 3 moles par litre de milieu réactionnel et par heure est tout à fait recommandé.

La pression d'hydrogène est avantageusement comprise entre 1 bar (0,1 MPa) et 100 bars (10 MPa) et de préférence entre 5 bars (0,5 MPa) et 25 bars (2,5 MPa).

La présente invention sera plus complètement décrite à l'aide des exemples suivants qui ne doivent en aucun cas être considérés comme limitatifs de l'invention.

Exemples 1 et 2 : Hydrogénation du 3,4-dichloronitrobenzène

Dans un autoclave en acier inox, on charge 90 ml de méthanol et 10 ml d'eau, la quantité de catalyseur

EP 0 421 878 A1

indiquée dans le tableau 1. Après fermeture, le réacteur est purgé plusieurs fois avec de l'azote puis de l'hydrogène.

La pression est fixée à 18 bars (1,8 MPa). Le mélange réactionnel est chauffé à 75° C. Dès que cette température est atteinte, on procède à l'injection en 30 minutes du substrat (25 g de dichloronitrobenzène dissous dans 135 ml de méthanol).

En fin de réaction, le réacteur est refroidi à température ambiante puis dégazé.

La masse réactionnelle est filtrée et le filtrat analysé par chromatographie en phase gazeuse.

On dose par le nitrate d'argent les ions chlorures (Cl⁻) présents dans le filtrat ; ces ions chlorures représentent l'hydrodéchloration du 3,4-dichloronitrobenzène qui s'est produite pendant l'hydrogénation.

TABLEAU 1

| Catalyseur | | | TT % 3,4-DCNB | RR % | | | Cl⁻ % mol DCNB |
|---|---|---|---|---|---|---|---|
| EX | Nature et quantité de métal en % | masse (g) | | 3,4-DCA | 3-CA | A | |
| 1 | 0,5 Pt/Al$_2$O$_3$ | 0,7 | 100 | 99,5 | 0,25 | <0,1 | 1,1 |
| 2 | 0,1 Pt/Al$_2$O$_3$ | 1,4 | 100 | 99,5 | 0,2 | <0,1 | 0,5 |

TT = Taux de transformation

RR = Rendement par rapport au substrat (3,4-DCNB) engagé.

3,4-DCNB = 3,4-dichloronitrobenzène

3,4-DCA = 3,4-dichloroaniline

3-CA = 3-chloroaniline

A = aniline

Pour des taux de transformation de 100 %, on note une hydrodéchloration très faible par rapport au 3,4-DCNB engagé.

Exemples 3 à 13

Dans un réacteur de 750 cm$_3$ en acier inox, on introduit 270 ml de méthanol et 30 ml d'eau, la quantité de catalyseur à base de platine déposé sur alumine à 0,5 %, est indiquée dans les tableaux 2, 3 et 4.

On purge le réacteur à l'azote et à l'hydrogène puis on pressurise avec de l'hydrogène sous une pression de 18 bars (1,8 MPa). On chauffe à 60° C (tableau 2) 80° C (tableau 3) 100° C (tableau 4).

On injecte en 16 minutes 30 ml d'un mélange de 182,2 mmol de 3-chloro 4-fluoro nitrobenzène (CFNB) et de 72,8 mmol de 4-fluoronitrobenzène (pFNB).

Les dosages des différents composés sont effectués par chromatographie en phase gazeuse : CFNB, pFNB, CFA (chloro-3 fluoro-4 aniline), pFA (parafluoro aniline), A (aniline).

Les ions chlorures sont dosés par le nitrate d'argent.

4

EP 0 421 878 A1

TABLEAU 2

| Influence de la quantité de catalyseur à 60° C | | | | | |
|---|---|---|---|---|---|
| ESSAIS | CATALYSEUR (g) | TT % | | HDCL % | HDF % |
| | | CFNB | pFNB | | |
| 3 | 0,5 | 18 | 18 | 0 | 0 |
| 4 | 1,0 | 100 | 100 | 0 | 0 |
| 5 | 2,0 | 100 | 100 | 0 | 0 |
| 6 | 4,0 | 100 | 100 | 0 | 0 |
| HDCL = hydrodéchloration exprimée en moles % par rapport au CFNB chargé. | | | | | |
| HDF = hydrodéfluoration exprimée en moles % par rapport au pFNB chargé. | | | | | |

TABLEAU 3

| Influence de la quantité de catalyseur à 80° C | | | | | |
|---|---|---|---|---|---|
| ESSAIS | CATALYSEUR (g) | TT % | | HDCL % | HDF % |
| | | CFNB | pFNB | | |
| 7 | 0,5 | 100 | 100 | 0,5 | 0 |
| 8 | 1,0 | 98,5 | 100 | 1,2 | 0 |
| 9 | 2,0 | 99 | 100 | 1,2 | 0 |
| 10 | 4,0 | 100 | 100 | 1,2 | 0,2 |

TABLEAU 4

| Influence de la quantité de catalyseur à 100° C | | | | | |
|---|---|---|---|---|---|
| ESSAIS | CATALYSEUR (g) | TT % | | HDCL % | HDF % |
| | | CFNB | pFNB | | |
| 11 | 0,5 | 100 | 100 | 0,1 | 0 |
| 12 | 1,0 | 100 | 100 | 1,1 | 0 |
| 13 | 4,0 | 100 | 100 | 1,0 | 0,5 |

Exemple 14

Dans le réacteur utilisé pour réaliser les exemples 3 à 13 on charge :

5

- 250 ml de méthanol à 10 % d'eau (V/V)
- 1,25 g de catalyseur Pd à 0,5 %/Al$_2$O$_3$

On porte la température de la masse réactionnelle (après purge à l'azote puis à l'hydrogène) à 110° C et la pression d'hydrogène à 18 bars.

Lorsque ces conditions sont atteintes et stabilisées, on injecte à raison de 25 g/h une solution constituée par :
- 12,5 g d'un mélange parafluoronitrobenzène (pFNB) + orthofluoronitrobenzène (oFNB) (70/30) molaire et
- 12,5 g de méthanol-eau à 35-5 (V/V).

La durée d'injection est de 15 heures. Afin de maintenir l'activité de la masse catalytique au niveau initial, il est nécessaire de procéder à 2 ajouts de 0,125 g de catalyseur après 5 et 10 heures de réaction.

Le taux de transformation des nitrofluorobenzènes est complet et les rendements déterminés par l'analyse et par rapport à chacun des fluoronitrobenzènes sont :
- parafluoro aniline : 99,8 %.
- orthofluoro aniline : 99,8 %.

On détecte la formation de 0,2 % d'aniline.


**Revendications**

1. Procédé de préparation des dérivés halogénoaminoaromatiques caractérisé en ce que l'on met en présence un dérivé halogénonitroaromatique avec un catalyseur constitué par un métal choisi parmi le platine et du palladium déposé sur un support à base d'alumine et de l'hydrogène.

2. Procédé selon la revendication 1, caractérisé en ce que le dérivé halogénonitroaromatique répond à la formule (I)

(Z)$_q$ (Y)$_p$ (X)$_n$- Ar -NO$_2$

dans laquelle :
- Ar représente un dérivé aromatique mono, poly ou hétérocyclique éventuellement substitué par un groupe alkyle contenant 1 à 4 atomes de carbone
- X, Y, Z représentent chacun un halogène choisi parmi le fluor, le chlore et le brome
- n, p, q représentent chacun un nombre entier supérieur ou égal à 0 et inférieur ou égal à 5, la somme n + p + q représentant un nombre entier supérieur ou égal à 1.

3. Procédé selon la revendication 2, caractérisé en ce que Ar représente un radical aromatique monocyclique et X et Y représentent le chlore et/ou le fluor.

4. Procédé selon la revendication 1, caractérisé en ce que la réaction est réalisée dans un solvant choisi parmi les alcools, les dérivés aromatiques.

5. Procédé selon la revendication 7, caractérisé en ce que le solvant est le méthanol.

6. Procédé selon la revendication 1, caractérisé en ce que la quantité de catalyseur utilisée est comprise entre 1 et 20 g par litre de milieu réactionnel.

7. Procédé selon la revendication 1, caractérisé en ce que dans le catalyseur utilisé la quantité pondérale de métal par rapport au support à base d'alumine est comprise entre 0,1 et 1 %.

8. Procédé selon la revendication 1, caractérisé en ce que la température de la réaction est comprise entre 70° C et 150° C et de préférence entre 70° C et 100° C.

Office européen
des brevets

RAPPORT DE RECHERCHE
EUROPEENNE

Numéro de la demande

EP 90 40 2749

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | EP-A-0 073 105  (E.I. DU PONT DE NEMOURS AND CO.)<br>* Revendications *<br><br>– – – – – | 1-3,6-8 | C 07 C 209/36<br>C 07 C 211/52 |

DOMAINES TECHNIQUES
RECHERCHES (Int. Cl.5)

C 07 C 209/00

Le présent rapport de recherche a été établi pour toutes les revendications

| Lieu de la recherche | Date d'achèvement de la recherche | Examinateur |
|---|---|---|
| La Haye | 19 décembre 90 | SANCHEZ Y GARCIA J.M |

CATEGORIE DES DOCUMENTS CITES
X : particulièrement pertinent à lui seul
Y : particulièrement pertinent en combinaison avec un
   autre document de la même catégorie
A : arrière-plan technologique
O : divulgation non-écrite
P : document intercalaire
T : théorie ou principe à la base de l'invention

E : document de brevet antérieur, mais publié à la
   date de dépôt ou après cette date
D : cité dans la demande
L : cité pour d'autres raisons
........................................................................
& : membre de la même famille, document
   correspondant